# EUROPEAN PATENT APPLICATION

(11) **EP 3 457 116 A1**
(43) Date of publication of application: **20.03.2019**
(21) Application number: 17796133.1
(22) Date of filing: 09.05.2017
(51) Int. Cl.: G01N 21/359, G01N 33/50, A61B 5/00

(54) **TUMOR CELL DETECTION METHOD AND TUMOR CELL DETECTION DEVICE**

(30) Priority: 09.05.2016 JP 2016093841
(71) Applicant: Sumitomo Electric Industries, Ltd., Osaka-shi, Osaka 541-0041 (JP); Wakayama Medical University, Wakayama 641-8509 (JP)
(72) Inventor: SOGAWA, Ichiro, Yokohama-shi Kanagawa 244-8588 (JP); KIMURA, Akinori, Yokohama-shi Kanagawa 244-8588 (JP); KOH, Yasuhiro, Wakayama-shi Wakayama 641-8509 (JP)
(74) Representative: Boult Wade Tennant LLP
(86) International application number: PCT/JP2017/017529
(87) International publication number: WO 2017/195772

(57) **Abstract**

A tumor cell detection method includes an analysis step of determining whether or not a cell contained in a sample is a tumor cell by a statistical technique, machine learning, or pattern recognition on the basis of a spectroscopic spectrum of the cell obtained by measuring the cell contained in the sample.

## Description

### Technical Field

The present invention relates to a tumor cell detection method and a tumor cell detection device.

### Background Art

It is known that, in a solid cancer patient, tumor cells are released from primary tumor tissue and circulated in the blood. A number of cases have been hitherto reported in which, by separating and recovering the circulating tumor cells (CTCs) in the blood from the blood, prognosis of a patient, molecular biological characteristics of tumor, and a change in behavior of tumor before and after medical treatment can be grasped. As a method of optically analyzing cells, for example, methods described in Patent Literatures 1, 2, and the like are mentioned.

### Citation List

### Patent Literature

Patent Literature 1: Japanese Unexamined Patent Publication No. 2001-523334
Patent Literature 2: Japanese Unexamined Patent Publication No. H11-500832
Patent Literature 3: Japanese Unexamined Patent Publication No. 2012-22002

### Summary of Invention

### Technical Problem

However, in the existing detection method as described in Patent Literatures 1, 2, and the like, since cells are observed under a microscope, there is a high possibility that CTCs, which exist in an extremely small amount in the blood (one CTC with respect to 10⁸ to 10⁹ blood cells) may be overlooked. Further, as another detection technique, a biological technique using immune reaction with respect to a specific marker protein expressed on the surface of CTCs is used (Patent Literature 3). In this case, since detection capability is dependent on expression of the marker protein, only tumor cells in which the marker protein is expressed can be detected. Further, since aggregation of tumor cells are non-uniform, the expression amount of the marker protein is also known to be not certain, and detection capability is unstable. Furthermore, the marker protein is not expressed in tumor cells in which epithelial mesenchymal transition (EMT) occurs, or the expression amount thereof is small, so that detection is difficult. Furthermore, it is considered that a decrease in survival rate when recovered cells are cultured and a degradation in accuracy in DNA analysis or protein analysis occur.

The present invention is made in view of the foregoing and aims to provide a tumor cell detection method and a tumor cell detection device capable of detecting tumor cells from various cells contained in a sample in non-contact manner.

### Solution to Problem

The invention of the present application provides the following.
(1) A tumor cell detection method, including an analysis step of determining whether or not a cell contained in a sample is a tumor cell by a statistical technique, machine learning, or pattern recognition on the basis of a spectroscopic spectrum of the cell obtained by measuring the cell contained in the sample.
(2) A tumor cell detection device, including:
   a light source unit irradiating a cell contained in a sample with measurement light;
   a detection unit receiving transmitted light or reflected light from the cell which is emitted by irradiation of the measurement light from the light source unit to acquire a spectroscopic spectrum of the cell; and
   an analysis unit determining whether or not the cell is a tumor cell by a statistical technique, machine learning, or pattern recognition on the basis of the spectroscopic spectrum acquired in the detection unit.

### Advantageous Effects of Invention

According to the present invention, there are provided a tumor cell detection method and a tumor cell detection device capable of detecting tumor cells from various cells contained in a sample in a non-contact manner.

### Brief Description of Drawings

FIG. 1 is a schematic configuration diagram of a tumor cell detection device according to an embodiment.
FIG. 2 is a diagram describing a conveyance example of a target object in the tumor cell detection device according to the embodiment.
FIG. 3 is a diagram describing a tumor cell detection method.
FIG. 4 shows examples of spectroscopic spectra of a cultured cancer cell (tumor cell), an erythrocyte, and a lymphocyte in a wavelength band of near-infrared light.
FIG. 5 is a diagram showing an example in which spectroscopic spectra obtained from cells are classified by principal component analysis.
FIG. 6 is a diagram showing an example in which spectroscopic spectra obtained from cells are classified by principal component analysis.
FIG. 7 is a diagram showing an example in which spectroscopic spectra obtained from cells are classified by principal component analysis.
FIG. 8 is a diagram showing an example in which spectroscopic spectra obtained from cells are classified by principal component analysis.

### Description of Embodiments

### [Description of Embodiments of Invention of Present Application]

First, embodiments of the present invention will be listed and described.
(1) A tumor cell detection method of the present application includes an analysis step of determining whether or not a cell contained in a sample is a tumor cell by a statistical technique, machine learning, or pattern recognition on the basis of a spectroscopic spectrum of the cell obtained by measuring the cell contained in the sample.

According to the tumor cell detection method, whether or not a cell contained in a sample is a tumor cell can be determined by a statistical technique, machine learning, or pattern recognition on the basis of a spectroscopic spectrum of the cell contained in the sample. Therefore, tumor cells can be detected from various cells contained in the sample in a non-contact manner.

(2) In the tumor cell detection method described in the above (1) of the invention of the present application, the spectroscopic spectrum may be a spectrum in a wavelength band of near-infrared light.

By using the spectrum in the wavelength band of the near-infrared light as the spectroscopic spectrum of the cell contained in the sample, whether or not the cell contained in the sample is a tumor cell can be suitably determined.

(3) In the tumor cell detection method described in the above (1) or (2) of the invention of the present application, the cell may be a cell contained in the blood, and in the analysis step, a boundary condition for determining whether or not a cell is a tumor cell may be calculated by a statistical technique, machine learning, or pattern recognition on the basis of a plurality of spectroscopic spectra obtained by measuring tumor cells and other cells in the blood, and whether or not the cell is a tumor cell may be determined on the basis of the spectroscopic spectrum obtained by measuring the cell contained in the sample and the boundary condition.

By employing the configuration in which the boundary condition is obtained in advance as described above and whether or not the cell contained in the sample is a tumor cell is determined on the basis of the boundary condition, whether or not the cell contained in the sample is a tumor cell can be suitably determined.

(4) A tumor cell detection device of the present application includes: a light source unit irradiating a cell contained in a sample with measurement light; a detection unit receiving transmitted light or reflected light from the cell which is emitted by irradiation of the measurement light from the light source unit to acquire a spectroscopic spectrum of the cell; and an analysis unit determining whether or not the cell is a tumor cell by a statistical technique, machine learning, or pattern recognition on the basis of the spectroscopic spectrum acquired in the detection unit.

According to the tumor cell detection device, whether or not a cell contained in a sample is a tumor cell can be determined by a statistical technique, machine learning, or pattern recognition on the basis of a spectroscopic spectrum of the cell contained in the sample. Therefore, tumor cells can be detected from various cells contained in the sample in a non-contact manner.

(5) In the tumor cell detection device described in the above (4) of the invention of the present application, the detection unit may acquire a spectrum in a wavelength band of near-infrared light as the spectroscopic spectrum.

By using the spectrum in the wavelength band of the near-infrared light as the spectroscopic spectrum of the cell contained in the sample, whether or not the cell contained in the sample is a tumor cell can be suitably determined.

In the tumor cell detection device described in the above (4) or (5) of the invention of the present application, the cell may be a cell contained in the blood, and the analysis unit may calculate a boundary condition for determining whether or not a cell is a tumor cell by a statistical technique, machine learning, or pattern recognition on the basis of a plurality of spectroscopic spectra obtained by measuring tumor cells and other cells in the blood and may determine whether or not the cell is a tumor cell on the basis of the spectroscopic spectrum obtained by measuring the cell and the boundary condition.

By employing the configuration in which the boundary condition is obtained in advance as described above and whether or not the cell contained in the sample is a tumor cell is determined on the basis of the boundary condition, whether or not the cell contained in the sample is a tumor cell can be suitably determined.

(7) In the tumor cell detection device described in any one of the above (4) to (6) of the invention of the present application, the detection unit may include a dispersing means for dispersing transmitted light or reflected light from the cell, and the dispersing means may be a wavelength selection filter, an interference optical system, a diffraction grating, or a prism.

When the detection unit includes a dispersing means selected from a wavelength selection filter, an interference optical system, a diffraction grating, or a prism, the spectroscopic spectrum can be acquired in the detection unit while light from the cell contained in the sample is suitably dispersed, and thus options of a light source usable in the light source unit can be broadened.

### [Details of Embodiments of Invention of Present Application]

Specific examples of the tumor cell detection method and the tumor cell detection device according to the present invention will be described below with reference to the appended drawings. Incidentally, the present invention is not limited to these examples and is intended to include all modifications that are indicated by the scope of the claims and that are within the interpretation and the scope equivalent to the scope of the claims.

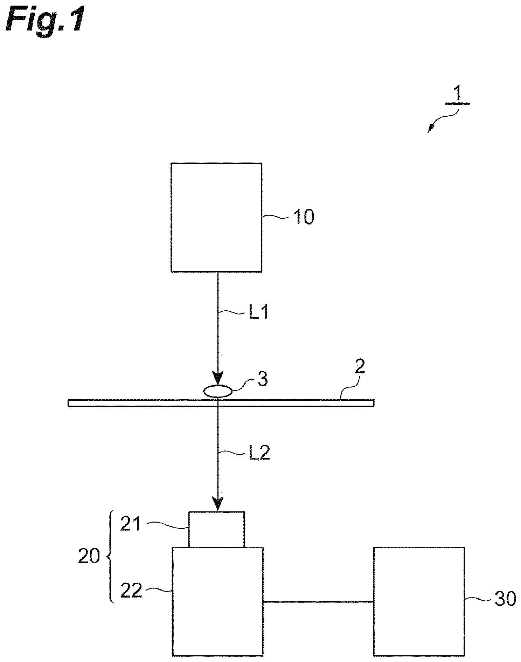

FIG. 1 is a schematic configuration diagram of a tumor cell detection device according to an embodiment of the present invention. As illustrated in FIG. 1, a tumor cell detection device 1 aims mainly to determine whether or not a cell as a target object 3 on a measurement table 2 is a tumor cell on the basis of optical measurement.

The tumor cell detection device 1 according to the present embodiment aims to detect tumor cells circulating in the blood. Therefore, one which becomes the target object 3 is a cell existing in the blood. Further, in the present embodiment, description will be given to a case where tumor cells as detection targets are circulating tumor cells (CTCs) in the blood, but other cells such as circulating endothelial cells (CECs) or circulating endothelial progenitors (CEPs) can be used as detection targets. Incidentally, in a case where cells in the blood are directly evaluated, the sample is blood. Further, a cell as the target object 3 may be in a state of being extracted from the blood, or the detection by the tumor cell detection device 1 may be performed in a state where the target objects 3 are dispersed in the blood or a blood-derived liquid. In a case where the target objects 3 are dispersed in a liquid different from the blood, the sample is a liquid in which the cells as the target objects 3 are dispersed. In FIG. 1, an example in which the cell of the target object 3 is placed on the measurement table 2 is illustrated.

In the tumor cell detection device 1, the spectrum of transmitted light obtained by irradiating the target object 3 with measurement light is measured, and whether or not the target object 3 is a tumor cell is determined on the basis of the spectrum. For this reason, the tumor cell detection device 1 includes a light source unit 10, a detection unit 20, and an analysis unit 30.

The light source unit 10 irradiates a region on which the target object 3 is placed with measurement light L1. As a light source of the light source unit 10, a halogen lamp or the like can be used. Further, it is also possible to use, as the light source of the light source unit 10, a SC light source that includes a seed light source and a non-linear medium, inputs light emitted from the seed light source to the non-linear medium, expands a spectrum to a wide band by a non-linear optical effect in the non-linear medium, and outputs the light as supercontinuum (SC) light. In the case of using the SC light source as the light source of the light source unit 10, since heating by the SC light source is reduced as compared to a halogen lamp, influence on the cell as the target object 3 can be reduced. Further, the light source unit 10 may have a function of modulating the intensity.

Incidentally, the wavelength of the measurement light L1 irradiated by the light source unit 10 in the present embodiment is not particularly limited, but the wavelength is appropriately selected depending on the target object 3, a liquid near the target object 3, and the like. As the measurement light LI, near-infrared light can be used. The near-infrared light is light of a wavelength band having a wavelength range of 800 nm to 2500 nm. Incidentally, as the measurement light L1, visible light can also be used. The visible light is light of a wavelength band having a wavelength range of 400 nm to 800 nm. In addition, the near-infrared light and the visible light may be combined and used as the measurement light L1.

The detection unit 20 receives light obtained by diffusely reflecting the measurement light L1 emitted from the light source unit 10 on the surface of the target object 3 or light output to a direction in which the detection unit 20 is disposed after the measurement light L1 passes through the target object 3, and detects the light as the spectroscopic spectrum of the target objects 3. Incidentally, the spectroscopic spectrum in the present embodiment refers to a series of data obtained by extracting an intensity value in an arbitrary wavelength from spectral information and paring the intensity value with the corresponding wavelength. Five or more combinations of a wavelength and an intensity value are contained in the spectroscopic spectrum, but in a case where there are many pieces of information of the combinations of a wavelength and an intensity value, analysis accuracy described later is improved. Further, the spectral information is a group of light intensity information in an arbitrary wavelength. Specific examples thereof include a reflected light intensity, a transmitted light intensity, and absorbance. Therefore, those obtained by arranging the reflected light intensity, the transmitted light intensity, and the absorbance in the wavelength order are a reflected light spectrum, a transmitted light spectrum, and an absorbance spectrum. The detection unit 20 acquires any of the reflected light spectrum, the transmitted light spectrum, and the absorbance spectrum as a spectroscopic spectrum. Incidentally, the reflected light includes diffusely reflected light, directly reflected light, and the like.

The detection unit 20 includes a spectroscope 21 (dispersing means) and a detector 22. The spectroscope 21 of the detection unit 20 has a function of dispersing light entering as transmitted light L2 to wavelength light. As the spectroscope 21, for example, a wavelength selection filter, an interference optical system, a diffraction grating, or a prism can be used. In a case where the spectroscope 21 is configured by being selected from the above-described means, the spectroscopic spectrum can be acquired in the detection unit 20 while light from the target object 3 is suitably dispersed, and thus options of a light source usable in the light source unit 10 can be broadened.

As the detector 22 of the detection unit 20, for example, an MCT detector formed of mercury, cadmium, and tellurium, an InGaAs detector, or the like can be used. In the present embodiment, a configuration is illustrated in which the transmitted light L2 from the target object 3 is received and dispersed by the spectroscope 21, and then the transmitted light spectrum is acquired as the spectroscopic spectrum of the target objects 3 in the detector 22. In this case, the detection unit 20 is provided at a position facing the light source unit 10 with the target object 3 interposed therebetween. Incidentally, in a case where the diffusely reflected light spectrum from the target object 3 is acquired as the spectroscopic spectrum, the light source unit 10 and the detection unit 20 are provided at the same side with respect to the target object 3. Information of the spectroscopic spectrum detected by the detection unit 20 is transmitted to the analysis unit 30.

Further, the detection unit 20 may be a hyperspectral sensor that acquires a hyperspectral image. The hyperspectral image is an image in which one pixel is configured by N pieces of wavelength data, and includes spectral information constituted of reflection intensity data corresponding to each of a plurality of wavelengths per pixel. That is, the hyperspectral image is characterized by having a plurality of pieces of wavelength intensity data for each of the pixels constituting the image and is thus three-dimensionally-configured data having both a two-dimensional element as an image and an element as spectral data. Incidentally, in the present embodiment, the hyperspectral image refers to an image configured by pixels having intensity data in at least five wavelength bands per one pixel.

Incidentally, in the present embodiment, although the configuration in which the spectroscopic spectrum is acquired in the detection unit 20 while the transmitted light L2 from the target object 3 is dispersed has been described, the configuration for acquiring the spectroscopic spectrum in the detection unit 20 is not limited to the above description. For example, a configuration in which a wavelength of light emitted from the light source of the light source unit 10 is variable may be employed. In this case, since the wavelength of the measurement light L1 varies, the spectroscopic spectrum of the cell as the target object 3 can be acquired in the detection unit 20 by sequentially detecting the transmitted light L2 emitted from the cell as the target object 3 in response to a change in wavelength of the measurement light L1. In this way, the configurations of the light source unit 10 and the detection unit 20 for acquiring the spectroscopic spectrum of the cell as the target object 3 can be appropriately changed.

The analysis unit 30 has a function of determining whether or not the target object 3 is a tumor cell by receiving spectroscopic spectrum information of the target object 3 transmitted from the detection unit 20 and performing arithmetic processing or the like. The analysis unit 30 may perform derivation of an absorption spectrum, derivation of a second-order differential spectrum of a measurement spectrum, derivation of a second-order differential spectrum of an absorption spectrum, and the like.

The analysis unit 30 is configured as a computer including hardware such as a central processing unit (CPU), a random access memory (RAM) and a read only memory (ROM) which are main storage devices, a communication module performing communication with other devices such as a detection unit, and an auxiliary storage device such as a hard disk. Further, the function as the analysis unit 30 is exerted by these constituent elements being operated.

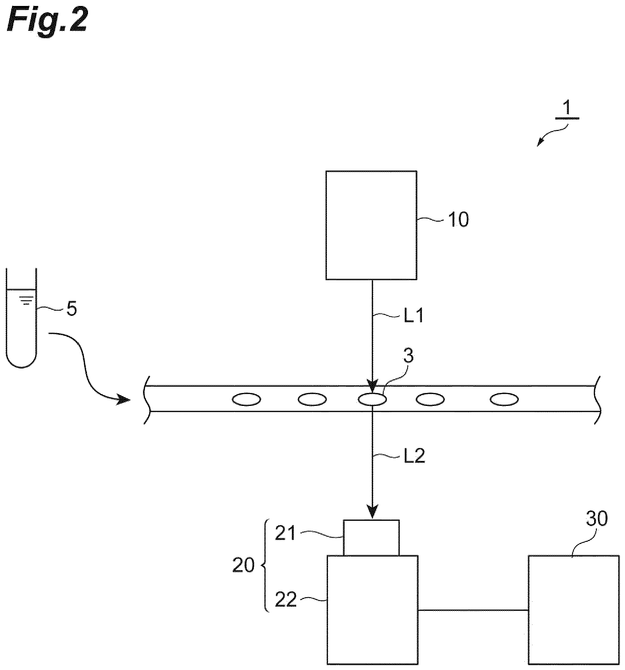

FIG. 2 is a diagram describing another configuration of the tumor cell detection device 1. In FIG. 2, detection is performed by the tumor cell detection device 1 in a state where the cell as the target object 3 is dispersed in the blood or a blood-derived liquid. In this case, a liquid containing the cell as the target object 3 (blood or a blood-derived liquid) is supplied from a sample tube 5 that accommodates the liquid containing the cell along a flow path 51 connected to the sample tube 5. When the inner diameter or the like of the flow path 51 is adjusted such that the cell as the target object 3 is in a state of being dispersed in the liquid, cells move one by one between the light source unit 10 and the detection unit 20 along the flow path 51. Therefore, in the tumor cell detection device 1, measurement of the cell in the liquid can be independently performed.

In the case of the configuration illustrated in FIG. 2, the transmitted light L2 emitted from the target object 3 is detected in the detection unit 20 by irradiating the target object 3 in the liquid flowing along the flow path 51 with the measurement light L1. In this way, the configuration for irradiating the target object 3 with the measurement light L1 and the configuration for detecting the transmitted light L2 from the target object 3 can be appropriately changed. Further, in the tumor cell detection device 1, a configuration in which measurement of a plurality of cells is simultaneously performed can also be employed. In this case, in the detection unit 20, a configuration in which spectroscopic spectra of a plurality of cells are simultaneously acquired can also be employed. However, determination on whether or not the cell as the target object 3 is a tumor cell is performed in a cell unit. In this way, the configurations of the light source unit 10 and the detection unit 20 can be appropriately changed.

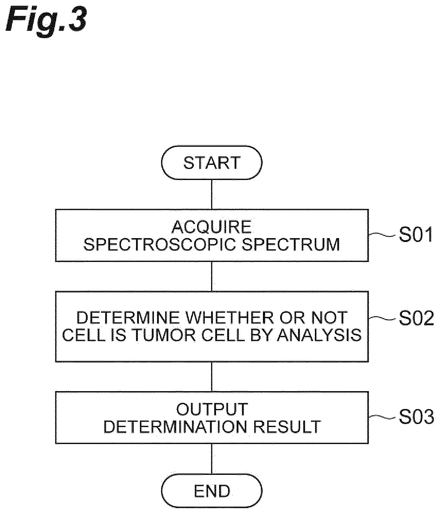

Next, a test method by the tumor cell detection device 1 will be described with reference to FIG. 3. In the tumor cell detection device 1, the test method includes: a step of acquiring, as the spectroscopic spectrum of the target objects 3, a diffusely reflection spectrum or transmission spectrum of a cell by irradiating the cell as the target object 3 of the test with the measurement light L1 (S01); a step of determining whether or not the cell as the target object 3 is a tumor cell on the basis of the spectroscopic spectrum obtained in the acquisition step (S02: an analysis step); and a step of outputting the determination result (S03). In the step of acquiring a spectroscopic spectrum (S01), the measurement light L1 is emitted to the target object 3 from the light source unit 10. The measurement light L1 emitted from the light source unit 10 is incident to the target object 3. The transmitted light L2, which proceeds to the direction of the detection unit 20, of light transmitted to the target object 3 arrives the detection unit 20 and a transmission spectrum as the spectroscopic spectrum of the target object 3 is acquired. Further, in the step of determining whether or not the cell is a tumor cell (S02), a treatment for the cell as the target object 3 is performed on the basis of the spectroscopic spectrum (transmission spectrum) obtained by the detection unit 20 and transmitted to the analysis unit 30.

Herein, a tumor cell detection method by the tumor cell detection device 1 according to the present embodiment is characterized in that a statistical technique, machine learning, or pattern recognition is used when whether or not the cell as the target object 3 is a tumor cell is determined on the basis of the spectroscopic spectrum of the cell. This point will be described in detail.

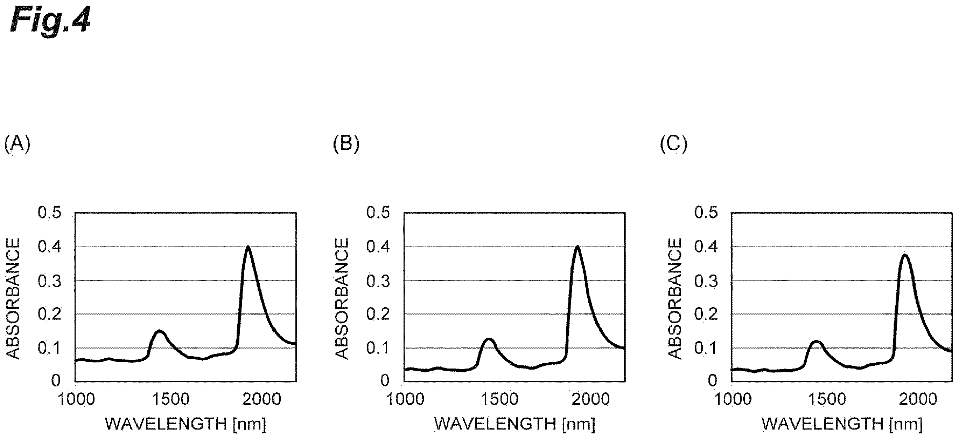

FIG. 4 shows examples of spectroscopic spectra of a cultured cancer cell (tumor cell), an erythrocyte, and a lymphocyte in a wavelength band of near-infrared light (1000 nm to 2200 nm). FIG. 4(A) shows a spectroscopic spectrum of a cultured cancer cell, FIG. 4(B) shows a spectroscopic spectrum of an erythrocyte, and FIG. 4(C) shows a spectroscopic spectrum of a lymphocyte (absorbance spectrum). Incidentally, each of the spectroscopic spectra shown in FIGS. 4(A) to 4(C) is a spectrum obtained by converting absorbance at each wavelength using a reference spectrum of a light quantity of 100% and a reference spectrum of a light quantity of 0% and then plotting the absorbance. The cultured cancer cell, the erythrocyte, and the lymphocyte are all cells contained in the blood, but as shown in FIGS. 4(A) to 4(C), these cells are difficult to classify on the basis of the shape of the spectroscopic spectrum, absorbance at a specific wavelength, and the like. On the other hand, in the tumor cell detection method according to the present embodiment, a tumor cell is separated from other cells by a statistical technique, machine learning, or pattern recognition.

As the statistical technique, for example, principal component analysis, factor analysis, or the like can be used. In addition, as the machine learning, for example, a support vector machine (SVM) or the like can be used. Further, as the pattern recognition, for example, an MT method or the like can be used.

In the present embodiment, as an analytical technique for specifying a tumor cell, a case where principal component analysis (PCA) is used will be described. Specifically, a plurality of absorbance spectra of three types of cells shown in FIGS. 4(A) to 4(C), that is, a cultured cancer cell (tumor cell), an erythrocyte, and a lymphocyte were acquired and it was checked whether a tumor cell can be specified on the basis of the principal component analysis using these absorbance spectra.

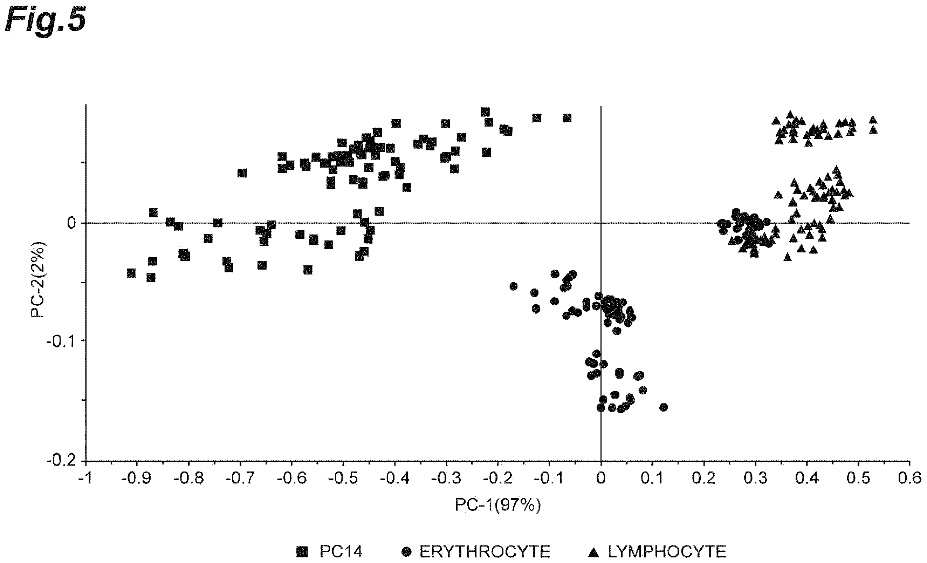

Specifically, by performing the principal component analysis based on the absorbance spectra of the cultured cancer cell (tumor cell), blood cells (erythrocyte and lymphocyte), a first principal component and a second principal component related to characteristics of each absorbance spectrum were obtained. Further, a score value of the first principal component and a score value of the second principal component in each absorbance spectrum were calculated and plotted. The result thereof is shown in FIG. 5.

When the score values of the first principal component and the second principal component obtained from each absorbance spectrum using the principal component analysis are plotted, as shown in FIG. 5, cultured cancer cells (PC14), erythrocytes, and lymphocytes can be classified into groups different from one another. Incidentally, in FIG 5, there is a part at which some of erythrocytes and lymphocytes are overlapped. However, it was possible to clearly discriminate cultured cancer cells (tumor cells) and blood cells (erythrocytes and lymphocytes). When tumor cells and blood cells can be clearly discriminated in this way, the boundary condition for classifying both the cells can be specified.

Therefore, when determination on whether or not a cell whose type is not known yet is a tumor cell is performed, as described above, the principal component analysis is performed using spectroscopic spectra of a plurality of cells whose type is already known, and the boundary condition for determining whether or not cells are tumor cells is obtained while the first principal component and the second principal component are specified. Thereafter, score values corresponding to the first principal component and the second principal component relating to a spectroscopic spectrum of a cell as a determination target are obtained, and determination on whether or not the cell is a tumor cell is performed by comparison between the score values and the boundary condition.

In this way, according to the tumor cell detection device 1 and the tumor cell detection method, determination on whether or not a cell as the target object is a tumor cell can be performed in a non-contact manner. That is, a tumor cell can be detected from a cell whose type is not known yet in a non-contact manner.

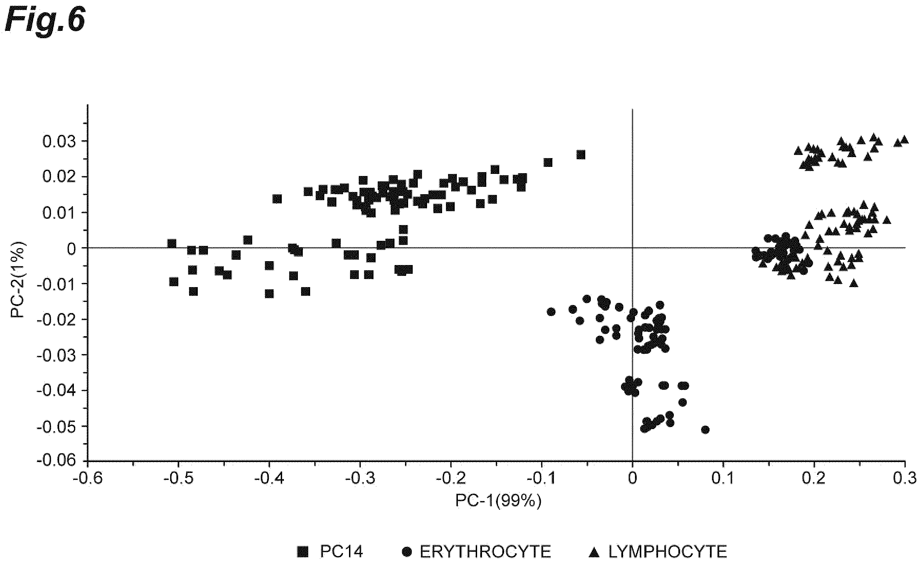

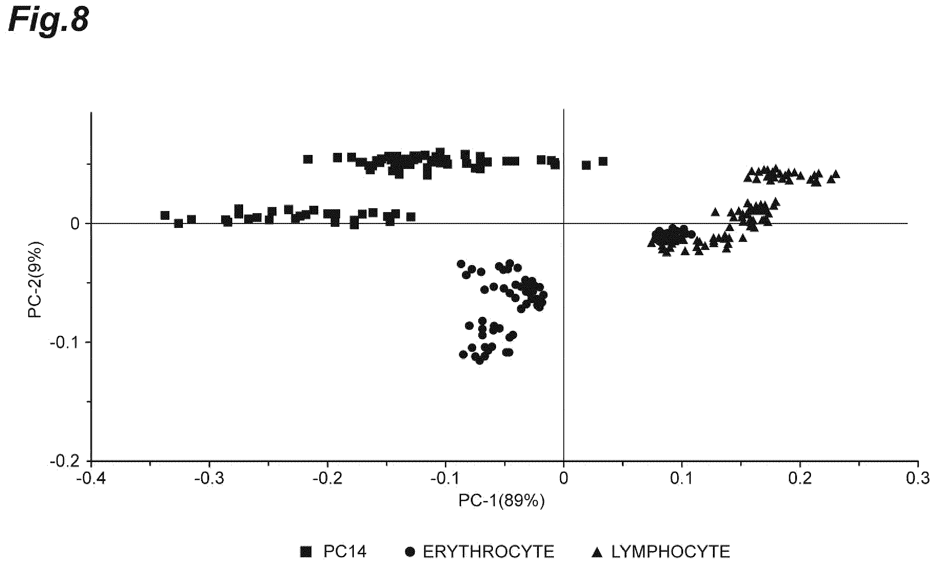

Incidentally, in FIG. 5, although an example in which cancer cells and blood cells are discriminated using a spectroscopic spectrum in a wavelength band of 1000 nm to 2200 nm has been described, even in the case of changing a wavelength band of the spectroscopic spectrum, cancer cells and blood cells can be discriminated using the principal component analysis. Other analysis examples are shown in FIGS. 6 to 8.

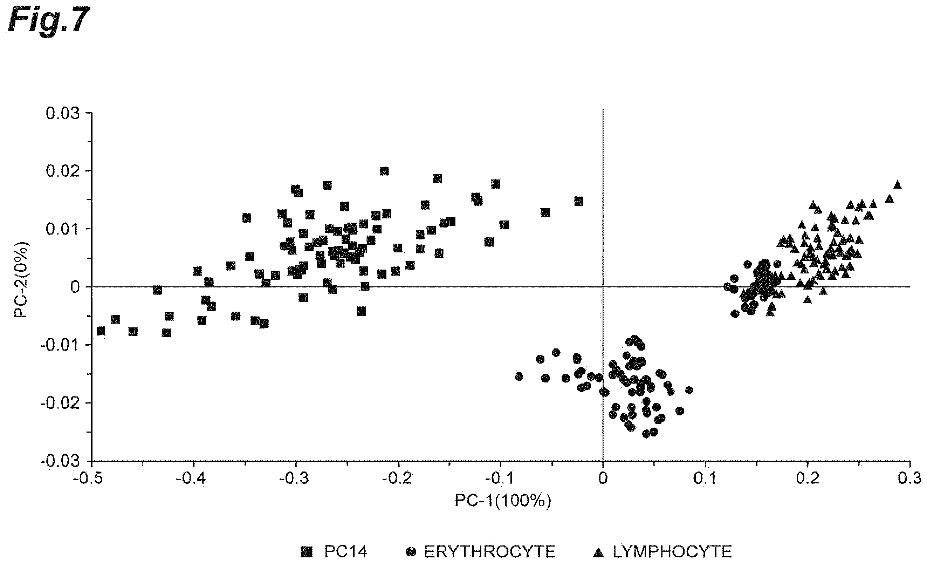

FIG. 6 shows an example in which cancer cells and blood cells are discriminated using a spectroscopic spectrum in a wavelength band of 1200 nm to 1500 nm. Further, FIG. 7 shows an example in which cancer cells and blood cells are discriminated using a spectroscopic spectrum in a wavelength band of 1500 nm to 1800 nm. Further, FIG. 6 shows an example in which cancer cells and blood cells are discriminated using a spectroscopic spectrum in a wavelength band of 1800 nm to 2100 nm. The spectroscopic spectra used in the analysis examples shown in FIGS. 6 to 8 are the same as the analysis example in FIG. 5, and are spectra in which a wavelength band to be analyzed is changed. Even in any of the analysis examples, it was possible to confirm that tumor cells and blood cells (erythrocytes and lymphocytes) are clearly discriminated. In this way, the wavelength band of the spectroscopic spectrum used when the principal component analysis is performed is not particularly limited and can be appropriately changed.

Further, detection of tumor cells by the tumor cell detection device 1 and the tumor cell detection method does not use absorbance or the like in a specific wavelength in the tumor cells but uses a difference in shape of spectroscopic spectra between tumor cells and other cells (blood cells). Therefore, although an example in which tumor cells are detected by performing the principal component analysis has been described in the above-described embodiment, detection of tumor cells can be performed even by using other techniques capable of performing classification based on a difference in shape of spectroscopic spectra, that is, a statistical technique, machine learning, or pattern recognition.

For example, in a case where cells are separated using a support vector machine in two groups of blood cells and cells other than blood cells, as spectroscopic spectra of cells whose type is already known, spectroscopic spectra of blood cells and tumor cells are acquired in advance. Then, a discrimination function and a discrimination threshold are created using the two types of spectroscopic spectra as teaching data and using a support vector machine. Thereafter, spectroscopic spectra of cells as analysis targets whose type is not known yet are acquired and analysis is performed. At this time, by comparing the result obtained by multiplying the spectroscopic spectra of cells as analysis targets by the discrimination function to the discrimination threshold, whether or not the cells are tumor cells can be determined. In this way, by using the spectroscopic spectra, tumor cells and blood cells can be separated using a statistical technique, machine learning, or pattern recognition.

Further, in the above description, although the analysis using a spectroscopic spectrum in a wavelength region of near-infrared light has been described, the shape of spectroscopic spectra between tumor cells and other cells vary also in other wavelength regions. Therefore, even in the case of using spectroscopic spectra with respect to light in other wavelength regions, similarly to the above-described example, tumor cells can be detected.

The function effects obtained by the tumor cell detection device 1 and the tumor cell detection method described above will be described. Conventionally, in order to detect tumor cells from various cells in the blood, tumor cells are necessary to dye with a specific marker protein. However, in the tumor cell detection device 1 and the tumor cell detection method described above, discrimination is realized by focusing on a difference in spectroscopic spectrum which is considered to occur mainly due to a difference in cell skeleton of both the blood cell and the tumor cell. That is, since the detection is not dependent on a specific marker protein, all tumor cells can be theoretically detected.

Further, since an antibody is used in the conventional technique, detection capability is dependent on the expression amount of the marker protein in cells as targets. For this reason, it was obvious that detection is difficult depending on the type of cells. On the other hand, in the tumor cell detection device 1 and the tumor cell detection method described above, a blood cell and a tumor cell are discriminated using a difference in spectroscopic spectrum therebetween.

For example, the cultured cancer cells (PC14) utilized in acquisition of the spectroscopic spectra used in the analysis examples of FIGS. 5 to 8 described above are cell lines with less expression amount of a marker protein, which is utilized in detection using an antibody in a device having been hitherto used, of cultured cell lines. That is, the cultured cancer cells (PC 14) are cells which are difficult to detect by the conventional technique. However, by using the tumor cell detection device 1 and the tumor cell detection method described above, it was confirmed that the cultured cancer cell can be separated from a blood cell regardless of the expression amount of the marker protein. In this way, in the tumor cell detection device 1 and the tumor cell detection method described above, detection accuracy is considered to be improved regardless of the type of tumor cells.

Further, in the tumor cell detection device 1 and the tumor cell detection method described above, since tumor cells can be detected only by acquiring spectroscopic spectra, tumor cells can be detected in a non-contact and non-invasive manner. Therefore, since a labeled substance such as a marker protein is not necessary attached to cells, cells with less damage can be used in the subsequent inspection, analysis, and research.

When tumor cells can be detected in a non-contact manner, it is expected that DNA analysis or protein analysis using tumor cells is greatly facilitated without being limited to detection of the number of tumor cells which is conventionally performed. Further, by using the technique described in the embodiment above, it is considered that data of tumor cells with higher reliability than that in the related art can be obtained. Therefore, as alternative diagnosis for diagnosis using a tumor tissue that is a standard in cancer diagnosis in practical clinic, possibility of clinical application of diagnosis using tumor cells in the blood such as CTCs is expected to be broadened. Further, when tumor cells are easily detected from cells in the blood, development of cancer basic research using the recovered tumor cells, identification of a novel therapeutical target as the result thereof, and the like are also expected. In a case where the tumor cell detection device 1 and the tumor cell detection method described above are put to practical use as research and medical equipment, since the tumor cell detection device 1 and the tumor cell detection method can be expected to be introduced to research institutions performing researches and foundation hospitals in medical care for cancer, it is considered that a large economic effect can be expected.

Incidentally, the tumor cell detection device 1 and the tumor cell detection method according to the present invention are not limited to the above-described embodiments. For example, there is no limitation on the configuration in which the tumor cell detection device 1 includes the light source unit 10, the detection unit 20, and the analysis unit 30 as described in the above-described embodiment, and the configuration can be appropriately changed. Further, the tumor cell detection method can also be performed in a device not including the light source unit 10 and the detection unit 20 unlike the tumor cell detection device 1. That is, a configuration in which analysis is performed using spectroscopic spectra acquired in another device may be employed.

### Reference Signs List

1: tumor cell detection device, 2: measurement table, 3: target object, 5: sample tube, 10: light source unit, 20: detection unit, 30: analysis unit.

## Claims

1. A tumor cell detection method, comprising an analysis step of determining whether or not a cell contained in a sample is a tumor cell by a statistical technique, machine learning, or pattern recognition on the basis of a spectroscopic spectrum of the cell obtained by measuring the cell contained in the sample.

2. The tumor cell detection method according to claim 1, wherein the spectroscopic spectrum is a spectrum in a wavelength band of near-infrared light.

3. The tumor cell detection method according to claim 1 or 2,
wherein the cell is a cell contained in the blood, and
in the analysis step, a boundary condition for determining whether or not a cell is a tumor cell is calculated by a statistical technique, machine learning, or pattern recognition on the basis of a plurality of spectroscopic spectra obtained by measuring tumor cells and other cells in the blood, and whether or not the cell is a tumor cell is determined on the basis of the spectroscopic spectrum obtained by measuring the cell and the boundary condition.

4. A tumor cell detection device, comprising:
a light source unit irradiating a cell contained in a sample with measurement light;
a detection unit receiving transmitted light or reflected light from the cell which is emitted by irradiation of the measurement light from the light source unit to acquire a spectroscopic spectrum of the cell; and
an analysis unit determining whether or not the cell is a tumor cell by a statistical technique, machine learning, or pattern recognition on the basis of the spectroscopic spectrum acquired in the detection unit.

5. The tumor cell detection device according to claim 4, wherein the detection unit acquires a spectrum in a wavelength band of near-infrared light as the spectroscopic spectrum.

6. The tumor cell detection device according to claim 4 or 5,
wherein the cell is a cell contained in the blood, and
the analysis unit calculates a boundary condition for determining whether or not a cell is a tumor cell by a statistical technique, machine learning, or pattern recognition on the basis of a plurality of spectroscopic spectra obtained by measuring tumor cells and other cells in the blood and determines whether or not the cell is a tumor cell on the basis of the spectroscopic spectrum obtained by measuring the cell contained in the sample and the boundary condition.

7. The tumor cell detection device according to any one of claims 4 to 6,
wherein the detection unit includes a dispersing means for dispersing transmitted light or reflected light from the cell, and
the dispersing means is a wavelength selection filter, an interference optical system, a diffraction grating, or a prism.
